# FASCICULE DE BREVET EUROPEEN

(11) **EP 3 359 520 B1**
(45) Date de publication et mention de la délivrance du brevet: **10.07.2024**
(21) Numéro de dépôt: 16788151.5
(22) Date de dépôt: 26.09.2016
(51) Int. Cl.: C07C 51/44, C07C 57/04, C07C 51/46, C07C 51/487, C08F 20/06

(54) **PROCÉDÉ AMÉLIORÉ DE PRODUCTION D'ACIDE ACRYLIQUE DE GRADE POLYMÈRE**
VERBESSERTES VERFAHREN ZUR HERSTELLUNG VON POLYMERGRAD ACRYLSÄURE
IMPROVED PROCESS FOR PRODUCING POLYMER-GRADE ACRYLIC ACID

(30) Priorité: 06.10.2015 FR 1559493
(43) Date de publication de la demande: 15.08.2018
(73) Titulaire: Arkema France, 92700 Colombes (FR)
(72) Inventeur: FAUCONET, Michel, 57730 Valmont (FR); JAIN, Sandeep, 75003 Paris (FR)
(74) Mandataire: Arkema Patent
(86) Numéro de dépôt international: PCT/FR2016/052434
(87) Numéro de publication internationale: WO 2017/060583

(56) Documents cités:
- EP-A2- 2 066 613
- JP-A- 2002 179 617
- US-A- 5 759 358
- US-A1- 2004 015 014
- US-A1- 2004 267 050

## Description

### DOMAINE TECHNIQUE

La présente invention concerne la production d'acide acrylique de grade polymère. Le terme « grade polymère » indique que l'acide acrylique répond à des critères de haute qualité permettant son utilisation dans la fabrication de polymères acryliques notamment pour le domaine des super absorbants. Ce grade est aussi connu sous le terme d'acide acrylique glacial AAg.

L'invention a plus particulièrement pour objet un procédé de récupération/purification d'acide acrylique de grade polymère à partir d'un mélange réactionnel brut comprenant de l'acide acrylique, ledit procédé n'utilisant pas de solvant organique ni de traitement par cristallisation, et incluant un traitement à l'aide d'un agent chimique visant à réduire le taux d'aldéhydes résiduels.

L'invention a trait également à une installation adaptée pour la mise en oeuvre dudit procédé de récupération/purification, ainsi qu'à un procédé de production d'acide acrylique de grade polymère.

### ARRIERE-PLAN TECHNIQUE ET PROBLEME TECHNIQUE

L'acide acrylique est destiné essentiellement à la mise en oeuvre par les industriels de procédés de polymérisation (ou copolymérisation en présence d'autres monomères polymérisables), soit de l'acide acrylique tel quel, soit de ses dérivés sous forme de sels (par exemple acrylate de sodium), esters (acrylates d'alkyle) ou amides (acrylamide). Ces procédés sont conduits sous différentes formes, en masse, en solution, en suspension ou en émulsion.

Les procédés de polymérisation et/ou de copolymérisation mettant en jeu l'acide acrylique peuvent être très sensibles à la présence dans la charge de certaines impuretés, comme les composés insaturés ou les aldéhydes, qui peuvent parfois empêcher d'obtenir la valeur d'usage attendue, par exemple en limitant la conversion du monomère en polymère, en limitant la longueur de chaîne du polymère, en provoquant des réactions de réticulation ou en modifiant la réactivité de la réaction.

Certaines de ces impuretés, comme les composés saturés non polymérisables, peuvent aussi être particulièrement gênantes dans l'application finale du polymère, en modifiant les propriétés du polymère, en conférant des propriétés toxiques ou corrosives au polymère, ou encore en augmentant les rejets organiques polluants lors des étapes de fabrication du polymère et/ou du produit fini.

En conséquence, les utilisateurs d'acide acrylique ou de dérivés d'acide acrylique se montrent exigeants en matière de spécifications de qualité de l'acide acrylique. Celui-ci doit répondre à des seuils sévères en matière d'impuretés.

En outre, ces utilisateurs mettent en oeuvre des recettes pour la production de leurs polymères qui sont adaptées à une qualité « standard » d'acide acrylique ou de ses dérivés. Une modification des recettes utilisées par ces utilisateurs, en vue de les adapter à une qualité différente d'acide acrylique présenterait des inconvénients importants pour ces sociétés utilisatrices.

La qualité (ou la pureté) de l'acide acrylique, c'est-à-dire sa teneur en différentes impuretés, jouant un grand rôle dans les processus de polymérisation, les industriels fabriquant cet acide acrylique ont été conduits à mettre en jeu toute une série d'étapes de purification afin d'obtenir cet acide acrylique « standard » qu'on appelle habituellement l'acide acrylique de grade polymère ou l'acide acrylique glacial (AAg). Cet AAg ne répond pas à des spécifications reconnues officiellement et ayant un caractère universel, mais signifie pour chaque industriel le niveau de pureté à atteindre pour pouvoir conduire avec succès sa polymérisation sous forme acide ou sous forme d'un dérivé et obtenir des polymères compatibles avec leurs utilisations finales.

Les procédés de récupération/purification d'acide acrylique décrits dans l'art antérieur sont très complexes quand il s'agit de produire de l'acide acrylique de grade polymère et présentent de nombreux inconvénients.

En effet, les procédés de synthèse de l'acide acrylique conduisent toujours à un milieu réactionnel gazeux constitué d'un mélange complexe d'impuretés, généralement classées selon leur faculté à être condensées en un mélange liquide ou absorbées dans un mélange liquide, ou classées selon leur température d'ébullition par rapport à celle de l'acide acrylique (composés légers, ou composés lourds).

Il s'ensuit que les procédés de purification utilisent habituellement un ensemble d'opérations pour séparer et récupérer l'acide acrylique contenu dans cet effluent gazeux.

Certaines de ces opérations mettent en oeuvre un ou plusieurs solvants organiques comme agents d'absorption (échanges gaz - liquide) et/ou comme agents d'extraction en milieu liquide (échanges liquide - liquide), et/ou encore comme agents de séparation par distillation azéotropique. Ces procédés, qui incluent en particulier, en plus des étapes de séparation du produit désiré, des étapes de traitements de récupération et purification des solvants, mettent en jeu nécessairement un nombre important de colonnes de distillation conduisant à un acide acrylique technique, dans lequel la teneur en impuretés a été fortement réduite, mais ne satisfaisant pas les spécifications d'un acide acrylique glacial, nécessaires pour obtenir par exemple des polymères de haute masse moléculaire.

D'autres procédés, tel que par exemple celui décrit dans le brevet EP 2 066 613 B1, permettent de simplifier la récupération et la purification de l'acide acrylique, en réduisant de manière très significative le nombre d'opérations unitaires nécessaires à l'obtention du produit purifié. Ces procédés se caractérisent par le fait qu'ils ne mettent en jeu aucune addition de solvant organique extérieur au procédé, et qu'ils utilisent un nombre restreint de colonnes de distillation, en particulier inférieur à 3 colonnes. Ces procédés simplifiés permettent d'obtenir une qualité d'acide acrylique technique de pureté élevée, pouvant atteindre ou dépasser 99%, mais encore non suffisante pour les applications les plus exigeantes de l'acide acrylique nécessitant des polymères de hautes masses moléculaires, pour lesquelles une qualité d'acide acrylique glacial (ou de grade polymère) est requise. Pour atteindre une qualité d'acide acrylique glacial, il est nécessaire d'éliminer jusqu'à un niveau très poussé (au maximum quelques ppm), certaines impuretés encore présentes dans l'acide acrylique technique obtenu selon les procédés précités.

Il s'agit notamment de certains aldéhydes, comme le furfuraldéhyde, le benzaldéhyde et l'acroléine, ou d'autres impuretés comme la protoanémonine, composé lourd généré au cours de la synthèse de l'acide acrylique, ou encore d'inhibiteurs de polymérisation non phénoliques comme la phénothiazine susceptibles d'avoir été introduits lors de la synthèse de l'acide (méth)acrylique.

Ces impuretés peuvent être éliminées par une étape de traitement supplémentaire par cristallisation fractionnée, comme décrit par exemple dans les documents US 6,448,439 ou EP 2 066 613 B1.

La cristallisation fractionnée est une technique de séparation bien connue. Elle peut être mise en oeuvre sous différentes formes, cristallisation dynamique, cristallisation statique ou cristallisation en suspension (US 5,504,247 ; US 5,831,124 ; US 6,482,981).

Cependant, la purification supplémentaire par cristallisation fractionnée est coûteuse en investissement et en énergie requise pour les phases de refroidissement et réchauffement successives.

C'est pourquoi, des procédés de purification complémentaires par distillation sont couramment utilisés pour l'élimination des impuretés de l'acide acrylique technique. Toutefois, cette élimination jusqu'à des teneurs extrêmement faibles de quelques ppm ne peut pas être obtenue de manière économique par une simple distillation, en raison d'une part de leurs concentrations élevées par rapport au seuil de leur sensibilité vis-à-vis de la réactivité de polymérisation et d'autre part de la volatilité de certains de ces composés, trop proche de celle de l'acide acrylique.

Pour ce faire, les industriels utilisent généralement des procédés combinant une purification par distillation et un traitement chimique à l'aide d'un réactif qui forme avec les aldéhydes des produits de réaction lourds plus facilement séparables de l'acide acrylique par distillation.

Parmi les réactifs utilisables, on peut employer par exemple des amines, des composés de la famille des hydrazines ou l'aminoguanidine, utilisés tels quels ou sous forme de leurs sels, ou en mélange (US 3,725,208 ; EP 270999 ; EP 685448).

Les traitements chimiques qui sont décrits dans l'art antérieur présentent tous l'inconvénient de générer de l'eau lors de la réaction des aldéhydes avec le réactif. La présence d'eau dans l'acide acrylique peut également être dommageable pour la fabrication de certains polymères.

Pour cette raison, il a été proposé dans le document JP 49-95,920 de réaliser ce traitement chimique pendant une étape de distillation visant à éliminer l'eau et les composés légers en tête, avant une étape de distillation de l'acide acrylique destinée à séparer les composés lourds.

Dans le procédé de purification décrit dans le document JP 2002-179617, le traitement chimique des aldéhydes est réalisé dans un mélangeur statique.

Ces différentes techniques combinant une purification de l'acide acrylique de grade technique par distillation et traitement chimique des aldéhydes sont décrites notamment pour la purification de l'acide acrylique technique obtenu par des procédés conventionnels mettant en jeu au moins un solvant organique externe. Elles ont le désavantage de nécessiter une section de purification complémentaire, avec au moins une colonne à distiller supplémentaire équipée de ses annexes (rebouilleur, condenseur, pompes, etc), ce qui a un impact négatif sur le coût de l'unité industrielle et également sur la consommation énergétique.

En outre, le traitement chimique des aldéhydes s'accompagne généralement de la formation de solides qui s'accumulent progressivement et nécessitent des nettoyages fréquents des équipements, impactant négativement la productivité de l'installation.

Selon le procédé de purification d'acide acrylique décrit dans le brevet EP 1110940 de Nippon Shokubai, il est nécessaire d'ajuster entre 3 et 100, le rapport de concentration du furfural à l'acroléine en poids présent dans l'acide acrylique à traiter, de façon à améliorer l'efficacité du traitement d'élimination des aldéhydes par un agent chimique de type hydrazine. Dans ces conditions, la quantité d'agent chimique à introduire est réduite et la formation de polymères est limitée.

Le procédé de purification d'acide acrylique décrit dans le document US 5759358 met en oeuvre un traitement chimique associant deux types de composés (amines de groupe A et amines de groupe B) afin d'éliminer les aldéhydes.

US 2004/267050 décrit un procédé de production d'acide méthacrylique sensiblement pur, sans adresser spécifiquement le problème de l'élimination des aldéhydes.

Il subsiste donc un besoin de disposer d'un procédé de récupération/purification d'acide acrylique conduisant à une qualité d'acide acrylique de grade polymère (ou glacial) qui soit simplifié, rapide et facile à mettre en oeuvre (comportant le moins d'étapes possibles), ne nécessitant pas l'intervention de solvant organique et/ou azéotropique, ou de technologie coûteuse en énergie ou polluante et qui en outre ne génère pas de formation de polymères encrassant les équipements.

Les inventeurs ont maintenant découvert que ce besoin pouvait être satisfait en mettant en oeuvre une section de purification d'un mélange réactionnel gazeux d'acide acrylique sans addition de solvant organique externe, et sans traitement par cristallisation, en particulier dans une installation comprenant une colonne de déshydratation, et une colonne de finition (ou colonne de purification) alimentée par une partie du flux de pied de la colonne de déshydratation, et incluant une étape de traitement des aldéhydes à l'aide d'un agent chimique, réalisée à l'intérieur de la section de purification susmentionnée, et permettant de conduire directement à une qualité d'acide acrylique glacial. Il est par ailleurs apparu aux inventeurs que cette invention pouvait s'appliquer à l'acide acrylique produits à partir de différentes sources, ainsi qu'à cet acide dérivé de matières premières renouvelables, qui est susceptible de poser les mêmes problèmes de purification.

### RESUME DE L'INVENTION

La présente invention concerne un procédé de récupération d'acide acrylique de grade polymère, en l'absence de solvant organique, à partir d'un mélange réactionnel gazeux comprenant de l'acide acrylique obtenu par oxydation en phase gazeuse d'un précurseur de l'acide acrylique, comprenant au moins les étapes suivantes :
i) on soumet le mélange réactionnel gazeux à une déshydratation sans utiliser de solvant azéotropique dans une première colonne de distillation dite colonne de déshydratation, conduisant à un flux de tête dont une partie au moins est condensée et renvoyée à la colonne de déshydratation sous forme de reflux, et à un flux de pied ;
ii) on soumet au moins en partie le flux de pied de colonne de déshydratation à une distillation dans une seconde colonne dite colonne de finition, conduisant à un flux de tête, et à un flux de pied contenant des composés lourds ;
iii) on récupère un flux d'acide acrylique purifié par soutirage latéral de la colonne de finition ;
caractérisé en ce qu'il est mis en oeuvre en présence d'un agent de traitement chimique des aldéhydes, ledit agent de traitement chimique des aldéhydes étant choisi parmi l'hydrazine et ses sels.

L'agent de traitement chimique des aldéhydes est introduit dans la colonne de finition et le flux d'acide acrylique purifié soutiré à l'étape iii) est un flux gazeux d'acide acrylique de grade polymère.

Le terme « solvant azéotropique » désigne tout solvant organique présentant la propriété de former un mélange azéotropique avec l'eau.

Le terme « non condensable » ou « incondensable » désigne les composés dont le point d'ébullition est inférieur à la température de 20°C sous la pression atmosphérique.

Le terme « léger », qualifiant les composés sous-produits, désigne les composés dont le point d'ébullition est inférieur à celui de l'acide acrylique sous la pression de travail considérée, et par analogie, le terme « lourd » désigne les composés dont le point d'ébullition est supérieur à celui de l'acide acrylique.

Le terme « agent de traitement chimique des aldéhydes » signifie un agent chimique permettant de réduire jusqu'à un niveau très faible le taux d'aldéhydes présents dans le milieu à traiter.

Le terme « grade polymère » indique que l'acide acrylique répond à des critères de haute qualité permettant son utilisation dans la fabrication de polymères acryliques de haute masse moléculaire.

Le procédé selon l'invention peut comprendre en outre d'autres étapes préliminaires, intermédiaires ou subséquentes pour autant qu'elles n'affectent pas négativement l'obtention d'acide acrylique de grade polymère.

Selon certains modes de réalisation particuliers, l'invention présente également une ou, de préférence, plusieurs des caractéristiques avantageuses énumérées ci-dessous :
- l'agent de traitement chimique des aldéhydes est introduit dans la colonne de finition par l'intermédiaire du flux d'alimentation de ladite colonne ;
- l'agent de traitement chimique des aldéhydes est introduit directement dans la colonne de finition au niveau d'une section située entre le niveau d'alimentation de ladite colonne et le niveau du plateau de soutirage latéral de l'acide acrylique purifié ;
- l'agent de traitement chimique des aldéhydes est introduit dans l'installation par l'intermédiaire d'un dispositif de mélange comprenant au moins une capacité assurant le mélange intime de l'agent chimique avec le flux à traiter et fournissant les conditions les plus appropriées pour un traitement efficace. Dans ce mode de fonctionnement, la capacité assure le mélange des réactifs avec le flux d'acide acrylique contenant les impuretés aldéhydiques à éliminer, et le traitement chimique peut être réalisé dans des conditions de température et de temps de résidence permettant une efficacité optimale ;
- la colonne de finition est une colonne de distillation classique ;
- la colonne de finition est une colonne à paroi séparatrice alimentée d'un côté de la paroi par le flux de pied de colonne de déshydratation et le soutirage latéral du flux d'acide acrylique purifié est réalisé en phase gazeuse ou liquide dans la section située de l'autre côté de la paroi séparatrice. Le flux de tête de colonne de finition est renvoyé pour partie en tête de cette colonne, afin d'assurer un reflux liquide dans la section délimitée par la paroi séparatrice comprenant le soutirage latéral.
- un second soutirage en phase gazeuse sur la colonne de finition est effectué à une position située en-dessous du soutirage latéral de l'acide acrylique purifié.

Selon un mode réalisation de l'invention, le précurseur de l'acide acrylique est l'acroléine.

Selon un mode réalisation de l'invention, l'acroléine est obtenue par oxydation de propylène ou par oxydéshydrogénation de propane.

Selon un mode réalisation de l'invention, le mélange réactionnel gazeux comprenant de l'acide acrylique obtenu par oxydation en phase gazeuse d'un précurseur de l'acide acrylique comprend du carbone d'origine renouvelable.

Selon un mode réalisation de l'invention, le précurseur de l'acide acrylique est dérivé du glycérol, de l'acide 3-hydroxypropionique ou de l'acide 2-hydroxypropionique (acide lactique).

Selon un mode réalisation préféré de l'invention, le mélange réactionnel gazeux comprend de l'acide acrylique dérivé de propylène obtenu selon un procédé d'oxydation en deux étapes.

Le procédé de récupération selon l'invention produit un flux d'acide acrylique de grade polymère, correspondant à une qualité satisfaisante pour produire des polymères de haute masse moléculaire, utilisables par exemple comme superabsorbants. Le procédé selon l'invention ne nécessite pas l'utilisation d'un solvant organique externe pour éliminer l'eau contenue dans le mélange réactionnel gazeux comprenant l'acide acrylique. Le procédé selon l'invention ne met pas en oeuvre de traitement par cristallisation. Dans un mode de fonctionnement préféré, le procédé selon l'invention ne nécessite à partir du mélange gazeux réactionnel qu'une section de purification à base de deux colonnes à distiller incluant une étape de traitement des aldéhydes à l'aide d'un agent chimique, réalisée à l'intérieur de ladite section.

Il est également décrit une installation pour récupérer de l'acide acrylique de grade polymère, adaptée pour mettre en oeuvre le procédé selon l'invention. L'installation comprend au moins :
a) une colonne de déshydratation ;
b) une colonne de finition connectée fluidiquement en pied de ladite colonne de déshydratation ;
c) éventuellement au moins une colonne de distillation connectée fluidiquement latéralement à ladite colonne de finition ;
d) un dispositif de mélange assurant l'introduction, le mélange et les conditions optimales de réaction de l'agent de traitement chimique dans le flux de pied de colonne de déshydratation alimentant la colonne de finition, comprenant éventuellement une capacité intermédiaire ;
e) au moins un système de soutirage latéral pour la colonne de finition.

Par « connexion fluidique » ou « connecté fluidiquement », on veut indiquer qu'il y a connexion par un système de conduites aptes à transporter un flux de matière. Ce système de connexion peut comprendre des vannes, des dérivations, des échangeurs de chaleur ou des compresseurs.

Par « capacité intermédiaire », on désigne une enceinte pouvant contenir un certain volume de liquide, alimentée par un flux liquide transitant intermédiairement dans cette capacité avant d'être envoyé dans l'étape suivante du procédé.

Par « dispositif de mélange », on entend un ensemble d'équipements en série assurant la dispersion la plus efficace de l'agent de traitement chimique dans le flux à traiter et les conditions de temps de séjour et température permettant d'obtenir une efficacité optimale de la réaction.

Un autre objet de l'invention est un procédé de production d'acide acrylique de grade polymère comprenant au moins les étapes suivantes :
A) on soumet à une oxydation en phase gazeuse au moins un précurseur d'acide acrylique pour former un mélange réactionnel gazeux comprenant de l'acide acrylique ;
B) on refroidit le mélange réactionnel gazeux ;
C) on soumet le mélange réactionnel gazeux refroidi au procédé de récupération de l'acide acrylique de grade polymère tel que défini précédemment.

La présente invention permet de produire de l'acide acrylique de grade polymère dans un procédé global ne mettant pas en oeuvre de solvant organique, réduisant ainsi les coûts énergétiques de récupération et les rejets par rapport à un procédé classique avec solvant organique. Le procédé selon l'invention met en oeuvre une technologie plus simple que la cristallisation fractionnée en mode discontinu ou que la cristallisation en continu en suspension.

Dans son mode de fonctionnement préféré, c'est-à-dire sans colonne à distiller supplémentaire par rapport au procédé de l'art antérieur produisant une qualité d'acide acrylique de grade technique, la présente invention permet en outre de réduire davantage les investissements et les coûts énergétiques de purification pour produire l'acide acrylique de grade polymère ou glacial. De plus, la formation de polymères solides encrassant les équipements étant évitée, l'invention apporte la possibilité de produire par campagne, sur la même installation, différentes qualités d'acide acrylique, selon la mise en oeuvre ou pas du traitement chimique des aldéhydes, ce qui assure une certaine souplesse opérationnelle pour les industriels.

D'autres caractéristiques et avantages de l'invention ressortiront mieux à la lecture de la description détaillée qui suit, en référence aux figures 1 à 2 annexées qui représentent :
- Figure 1 : Installation adaptée à la mise en oeuvre du procédé de récupération d'acide acrylique de grade polymère illustrant un mode de réalisation préféré de l'invention, utilisant une colonne de distillation classique comme colonne de finition.
- Figure 2 : Installation adaptée à la mise en oeuvre du procédé de récupération d'acide acrylique de grade polymère illustrant un deuxième mode de réalisation préféré de l'invention, utilisant une colonne de distillation à paroi séparatrice comme colonne de finition.

### DESCRIPTION DETAILLEE DE L'INVENTION

L'invention vise à produire de l'acide acrylique de haute pureté et est basée sur la mise en oeuvre d'un traitement à l'aide d'un agent chimique visant à éliminer jusqu'à un niveau très poussé (au maximum quelques ppm), certaines impuretés encore présentes dans l'acide acrylique technique obtenu selon un procédé n'utilisant pas de solvant organique externe. Il s'agit notamment d'aldéhydes dont la présence même à l'état de traces est préjudiciable pour l'obtention de polymères acryliques de haute masse moléculaire.

Dans la suite de l'exposé de l'invention, par « traitement chimique » on entend le traitement réalisé à l'aide de l'agent de traitement chimique des aldéhydes.

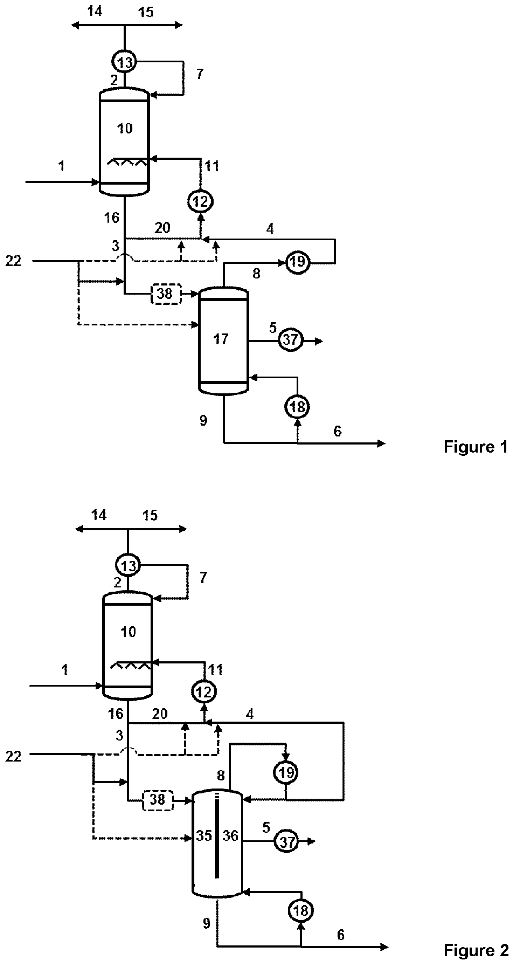

Selon un premier aspect de l'invention, le traitement chimique est intégré dans un procédé de récupération d'acide acrylique technique, conduisant ainsi directement à une qualité d'acide acrylique de grade polymère. Les modes de réalisation préférés sont représentés sur les Figures 1 et 2.

Selon un premier mode de réalisation de l'invention représenté à la Figure 1, un mélange réactionnel gazeux 1 comprenant de l'acide acrylique obtenu par oxydation en phase gazeuse d'un précurseur de l'acide acrylique alimente une première colonne de distillation 10. Le mélange réactionnel gazeux comportant un rapport massique eau/acide acrylique généralement compris entre 0,3 et 2 peut être préalablement refroidi avant d'être soumis à une déshydratation selon l'étape i) du procédé selon l'invention dans la colonne 10 dite colonne de déshydratation.

Le mélange réactionnel comprend en plus de l'eau et l'acide acrylique, des produits légers incondensables tels que l'azote, l'oxygène, le monoxyde et le dioxyde de carbone, ainsi que différents sous-produits légers ou lourds de différente nature chimique, pouvant être des aldéhydes légers comme l'acroléine, le formaldéhyde ou l'acétaldéhyde, des aldéhydes lourds tels que le furfuraldéhyde ou le benzaldéhyde, des acides légers tels que l'acide formique, l'acide acétique ou l'acide propionique, des acides lourds tels que l'acide maléique, l'acide benzoïque ou l'acide 2-butènoïque.

La colonne de déshydratation conduit à un flux de tête 2 dont au moins une partie est condensée dans un condenseur 13 et renvoyée à la colonne de déshydratation sous forme de reflux 7 pour absorber l'acide acrylique, l'autre partie 14/15 comprenant les composés légers incondensables étant généralement envoyée partiellement ou totalement à un dispositif d'épuration ou recyclée en partie vers d'autres étapes du procédé de production d'acide acrylique, de préférence dans une étape située en amont du réacteur de production du mélange réactionnel 1.

Selon un mode de réalisation, la totalité du flux de tête 2 de la colonne de déshydratation est envoyée dans le condenseur de tête 13.

Le but de l'étape i) de déshydratation est d'éliminer dans un flux de tête l'essentiel de l'eau présente dans le mélange réactionnel, mais aussi les composés légers incondensables et les composés légers condensables. La colonne de déshydratation fonctionne, au moins partiellement, comme une colonne de distillation. Elle est alimentée dans sa partie inférieure par le mélange réactionnel 1. Elle génère un flux de tête 2 comprenant l'essentiel de l'eau et des composés légers, et de l'acide acrylique et des composés lourds en quantité très faible, et un flux de pied 16 appauvri en composés légers comprenant l'essentiel de l'acide acrylique avec des sous-produits lourds.

La colonne de déshydratation comprend généralement de 5 à 50 plateaux théoriques, de préférence de 20 à 30 plateaux théoriques. Elle est équipée d'un échangeur de chaleur dont la fonction principale, dans la configuration représentée à la Figure 1, est, selon la température du gaz 1 entrant dans la colonne, de refroidir ou réchauffer le flux liquide 20 extrait du pied de colonne, avant de le renvoyer en reflux dans la partie inférieure de la colonne de déshydratation. Dans une autre configuration possible non représentée sur la Figure 1, le flux gazeux 1 est préalablement refroidi dans un échangeur externe avant de rentrer dans la colonne de déshydratation, et/ou le flux gazeux 1 est refroidi et condensé à reflux d'une partie du flux liquide 16, dans un échangeur de chaleur situé à l'extérieur de la colonne de déshydratation, avant d'être introduit dans cette colonne.

Avantageusement, la colonne de déshydratation fonctionne à la pression atmosphérique ou légèrement supérieure, jusqu'à une pression absolue de 1,5 10⁵ Pa. Avantageusement, la température dans la partie supérieure de la colonne de déshydratation est d'au moins 40°C, de préférence est comprise entre 40°C et 80°C. La température du flux de pied de la colonne de déshydratation ne dépasse pas de préférence 120°C.

Selon l'invention, l'essentiel de l'eau présente dans le mélange réactionnel gazeux comprenant l'acide acrylique est éliminé au cours de l'étape i) sans qu'il n'y ait de perte excessive d'acide acrylique dans le flux de tête 14/15.

Aucun solvant azéotropique n'est ajouté dans la colonne de déshydratation.

La teneur massique en eau dans le flux de pied de la colonne de déshydratation est généralement inférieure à 10%, de préférence inférieure à 7%. Une composition massique typique du flux de pied de la colonne de déshydratation comprend essentiellement de l'acide acrylique (84-90%), de l'acide acétique (5-10%), de l'eau (3-6%).

Selon l'étape ii) du procédé selon l'invention, le flux de pied 16 de la colonne de déshydratation est envoyé au moins en partie (flux 3), en tête d'une seconde colonne de distillation 17, dite colonne de purification ou colonne de finition, dans laquelle sont séparés un flux de tête 8 et un flux de pied 9.

Selon un mode de réalisation, une partie 20 du flux liquide 16 de pied de la colonne de déshydratation est envoyée dans un échangeur de chaleur 12 qui peut être un réchauffeur ou un refroidisseur et réinjectée dans la colonne de déshydratation, de façon à constituer une boucle de pied. De préférence, la partie 11 de la boucle de pied est réinjectée entre l'alimentation du mélange gazeux réactionnel et la tête de colonne de déshydratation. Le reste (flux 3) du flux liquide 16 est envoyé en alimentation de la colonne de finition 17. Selon une variante optionnelle de ce mode de réalisation, le flux 3 peut être stocké dans une capacité intermédiaire 38 avant d'être envoyé en alimentation de la colonne de finition 17. Dans cette option, il peut être avantageux de refroidir le flux 3 par un échangeur de chaleur situé avant la capacité intermédiaire, ou dans un échangeur situé sur un circuit de recirculation en boucle dans cette capacité, et le flux refroidi peut ensuite être réchauffé à travers un échangeur avant d'être introduit dans la colonne de purification 17.

Le flux gazeux de tête 8 de la colonne de purification est envoyé dans un condenseur 19. Le flux liquide sortant 4 est renvoyé vers la colonne de déshydratation, entre le pied et la tête de la colonne et de préférence au-dessus de l'alimentation du mélange gazeux réactionnel. Selon un mode de réalisation, comme représenté à la Figure 1, il est mélangé au flux de la boucle de pied de la colonne de déshydratation.

La colonne de finition 17 permet de séparer un flux de tête 8 comprenant de l'eau et les sous-produits légers condensables, un flux de pied 9 comprenant l'essentiel des sous-produits lourds, notamment des produits d'addition de Michael tels que l'acide 3-acryloxypropionique, de l'anhydride/acide maléique, de l'acide benzoïque, ainsi que des inhibiteurs de polymérisation, et un flux latéral 5 comprenant de l'acide acrylique purifié sous forme de liquide ou de vapeur, de préférence gazeux.

La colonne de finition 17 peut être une colonne de distillation classique comprenant généralement de 5 à 30 plateaux théoriques, de préférence de 8 à 20 plateaux théoriques. Cette colonne de distillation est associée à au moins un rebouilleur et un condenseur. La température et la pression dans la colonne 17 ne sont pas critiques, et peuvent être déterminées conformément aux méthodes de distillation connues de l'état de l'art. Cependant, de préférence, la colonne de finition 17 fonctionne à une pression inférieure à la pression atmosphérique, permettant de fonctionner à des températures relativement faibles, évitant ainsi la polymérisation des produits insaturés présents, et minimisant la formation de sous-produits lourds.

Avantageusement, la colonne de finition fonctionne sous une pression absolue allant de 5 kPa à environ 60 kPa, la température du flux de tête étant avantageusement comprise entre 40°C et environ 90°C, et la température du flux de pied étant comprise entre 60°C et 120°C.

En l'absence du traitement chimique selon l'invention, le flux 5 d'acide acrylique purifié, soutiré latéralement de la colonne de finition, correspond à un acide acrylique de grade technique.

Selon l'invention, un agent chimique 22 est introduit dans l'installation représentée à la Figure 1, afin de produire directement un acide acrylique de grade polymère. L'introduction peut être effectuée selon différentes façons :
Selon un premier mode de réalisation décrit sur la figure 1, on introduit l'agent de traitement chimique 22 dans la colonne de finition 17, soit au niveau d'une section située entre le niveau d'alimentation de ladite colonne et le niveau du plateau de soutirage latéral, soit préférentiellement par l'intermédiaire du flux 3 d'alimentation de ladite colonne.

Les produits lourds générés lors de la réaction de l'agent de traitement chimique avec les aldéhydes présents dans le flux alimentant la colonne de déshydratation sont éliminés d'abord dans le flux de pied 3 de la colonne de déshydratation, puis finalement dans le flux de pied 9 de la colonne de finition, avec les autres sous-produits lourds. L'eau produite par les réactions de formation de ces produits lourds est éliminée en partie en tête de la colonne de déshydratation, et en partie en tête de la colonne de finition. L'agent de traitement chimique est un réactif qui forme avec les aldéhydes présents dans le flux alimentant la colonne de finition des produits de réaction lourds plus facilement séparables de l'acide acrylique. Les réactions de formation de ces produits lourds produisent de l'eau qui est éliminée directement en tête de la colonne de finition, et les produits lourds formés sont éliminés dans le flux de pied 9 de la colonne de finition avec les autres sous-produits lourds.

En conséquence, un flux 5 gazeux est soutiré latéralement de la colonne de finition, qui, après condensation dans un échangeur 37, forme un flux liquide d'acide acrylique purifié exempt des aldéhydes, des composés lourds et des inhibiteurs de polymérisation non phénoliques et également débarrassé de l'eau. Il s'agit donc d'un flux d'acide acrylique débarrassé des impuretés gênantes pour sa polymérisation ultérieure, correspondant directement à une qualité d'acide acrylique de grade polymère ou glacial, qui peut être utilisée pour produire des grades de polymères les plus sensibles aux impuretés, comme par exemple les super absorbants, sans nécessiter de purification complémentaire, notamment par cristallisation fractionnée. Ce flux peut être ensuite additionné d'un inhibiteur de polymérisation.

Le flux de pied 9 de la colonne de finition 17 est renvoyé partiellement dans cette colonne à travers le rebouilleur 18. L'autre partie 6 peut être envoyée à une section de concentration (non représentée sur la Figure 1) en vue de récupérer l'acide acrylique monomère résiduel contenu dans le flux 6, et/ou envoyée dans une section de craquage en vue de régénérer l'acide acrylique à partir des composés lourds d'addition de Michael du type acide 3-acryloxypropionique, et/ou être utilisée comme matière première dans une unité de production d'esters acryliques.

Dans une variante, de manière optionnelle, on opère un deuxième soutirage latéral en phase gazeuse sur la colonne de finition 17 (non représenté sur la Figure 1), à une position située en dessous du soutirage latéral d'acide acrylique purifié de grade polymère. Ce flux concentré en acide acrylique, débarrassé de l'essentiel des produits lourds et produits de réaction de l'agent chimique avec les aldéhydes, peut être avantageusement valorisé, comme matière première d'un procédé d'estérification pour produire par exemple, sans limitation de choix, de l'acrylate de méthyle, de l'acrylate d'éthyle, de l'acrylate de butyle ou de l'acrylate de 2-éthylhexyle.

Dans cette variante, le deuxième flux soutiré latéralement correspond à un acide acrylique de qualité intermédiaire, en particulier de grade ester exempt des sous-produits lourds qui ont été concentrés dans le flux de pied 9 de la colonne de finition.

Selon un mode de réalisation préféré de l'invention (représenté sur la Figure 1), le mélange de l'agent de traitement chimique des aldéhydes avec le flux à traiter est réalisé en amont de la colonne de finition, dans un dispositif de mélange 38 permettant la dispersion la plus efficace de l'agent de traitement chimique dans le flux. Ce dispositif peut en particulier contenir en série une ou plusieurs capacités et un ou plusieurs équipements de mélange ou d'échange de chaleur, de façon à réaliser le traitement à une température et pendant un temps de séjour optimums. De manière non exhaustive, les équipements de mélange peuvent inclure des outils généralement utilisés par l'homme de l'art pour mélanger des liquides, comme des récipients agités ou recirculés ou des mélangeurs statiques, mais aussi tout type d'équipements permettant une dispersion rapide de l'agent chimique de traitement dans le flux à traiter, comme des mélangeurs à jet axial, à jet rotatif, éjecteurs à jet liquide, hydroéj ecteurs, pompes, filtres, etc.

Selon ce mode de réalisation, de façon préférentielle, le flux de pied de la colonne de déshydratation est refroidi de 60-120°C à 30-80°C préalablement ou en même temps que l'introduction de l'agent de traitement chimique des aldéhydes. Un dispositif possible est que le flux de pied de la colonne de déshydratation est refroidi et stocké intermédiairement dans un bac agité ou recirculé à travers une pompe et l'introduction de l'agent de traitement chimique des aldéhydes est réalisée dans ce bac. Le temps de séjour dans cette capacité intermédiaire est généralement de 5 à 120 min. Préalablement à l'alimentation de la colonne de finition par le mélange liquide provenant de cette capacité intermédiaire, le flux est de préférence réchauffé si nécessaire à une température de 70-90°C, dans un échangeur en ligne ou une capacité intermédiaire chauffée.

Ces différents modes de réalisation, et en particulier celui associant un dispositif de mélange, sont particulièrement avantageux car ils permettent de réduire la quantité d'agent de traitement chimique des aldéhydes nécessaire et réduisent l'encrassement des équipements en optimisant la réactivité de l'agent de traitement et en réduisant ainsi l'excès par rapport aux aldéhydes à éliminer.

Les agents de traitement chimique des aldéhydes utilisables dans l'invention sont des composés de la famille des hydrazines, tel que, sans limitation, l'hydrazine et ses sels, l'hydrate d'hydrazine, le sulfate d'hydrazine, les carboxylates d'hydrazine, le chlorhydrate d'hydrazine, la phénylhydrazine, la 4-nitrophénylhydrazine, et la 2,4-dinitrophénylhydrazine, seuls ou leurs mélanges en toutes proportions.

Les agents chimiques sont introduits tels quels dans le flux à traiter, ou en solution dans un solvant, par exemple en solution dans de l'acide acrylique.

L'agent chimique est introduit en quantité minimale pour obtenir une qualité d'acide acrylique glacial suffisamment débarrassée des impuretés aldéhydiques (en particulier acroléine, furfuraldéhyde et benzaldéhyde) pour répondre aux besoins des clients. En général, l'agent chimique est ajouté dans un rapport molaire de 0,5 à 10, de préférence de 1 à 5, par rapport à la totalité des aldéhydes présents dans le milieu à traiter.

En référence à la Figure 2 représentant une variante de l'invention, la colonne de finition 17 est remplacée par une colonne à paroi séparatrice comportant deux sections 35 et 36, la section 35 étant alimentée par le flux 3 de pied de la colonne de déshydratation, et le flux 5 d'acide acrylique de grade polymère étant soutiré latéralement à partir de la section 36.

En particulier, la paroi séparatrice n'est pas jointive avec la partie haute de la colonne ; ainsi les composés légers introduits par le flux 3 peuvent être facilement évacués dans le flux 4 en tête de colonne, après condensation du flux gazeux 8 dans le condenseur 19. Ces colonnes séparatrices sont notamment commercialisées par les sociétés Sulzer ou Montz.

Selon ce mode de réalisation, l'agent de traitement chimique 22 est introduit dans la section 35 de la colonne de finition soit par l'intermédiaire du flux 3 d'alimentation de ladite colonne soit au niveau d'une section située entre le niveau d'alimentation de ladite colonne dans la section 35 et le bas de cette section, selon les différentes manières décrites précédemment en relation avec une colonne classique.

Le flux de tête 8 de cette colonne de finition est condensé dans le condenseur 19 et renvoyé pour partie en tête de la colonne de finition afin d'assurer un reflux liquide dans la section 36 délimitée par la paroi séparatrice comprenant le soutirage latéral, l'autre partie 4 étant recyclée dans la boucle de pied de la colonne de déshydratation. Selon cette configuration, le soutirage latéral du flux d'acide acrylique de grade polymère est réalisé en phase gazeuse, puis est condensé dans un échangeur 37 et additionné d'inhibiteur de polymérisation tel que l'éther méthylique d'hydroquinone (EMHQ), à une teneur de 200 +/- 20 ppm. De manière avantageuse, un ou plusieurs inhibiteurs de polymérisation est (sont) introduit(s) dans le mélange liquide renvoyé en reflux en tête de colonne. En variante, le soutirage latéral du flux d'acide acrylique de grade polymère peut être réalisé en phase liquide, et le flux purifié est refroidi dans l'échangeur 37. Dans ce cas, de l'EMHQ est introduit dans le mélange liquide renvoyé en reflux en tête de colonne, en quantité telle que la concentration de cet inhibiteur dans le flux d'acide acrylique de grade polymère n'excède pas 220 ppm.

Il est apparu de façon surprenante que les problèmes de formation de solides n'apparaissent pas dans le procédé selon l'invention, alors que les rapports de concentration massique du furfural à l'acroléine présents dans les flux d'acide acrylique dans lesquels l'agent de traitement chimique est introduit, sont différents de ceux indiqués dans le brevet EP 1110940 permettant d'éviter la génération de polymères solides. Sans être liés par cette explication, les inventeurs pensent que l'agent de traitement chimique des aldéhydes est introduit de façon optimale dans un flux d'acide acrylique brut de composition mieux adaptée à la solubilisation des sous-produits de réaction (mode de réalisation préférentiel réalisé dans une installation ne comprenant que 2 colonnes de distillation) ou dans un flux d'acide acrylique technique de meilleure qualité que les grades techniques disponibles selon les procédés classiques de l'art antérieur (mode de réalisation avec traitement chimique de l'acide acrylique technique dans une section supplémentaire).

Le procédé selon l'invention dans toutes ses variantes permet de produire de l'acide acrylique glacial ou de grade polymère, ayant une teneur en poids en acide acrylique > 99 %, de préférence > 99,5 %, et comportant de préférence une teneur en aldéhydes totaux < 10 ppm, voire < 3 ppm.

L'acide acrylique de grade polymère obtenu selon le procédé de l'invention a également de préférence les teneurs en impuretés suivantes :
protoanémonine < 5 ppm, en particulier < 3 ppm
anhydride maléique < 100 ppm, en particulier < 50 ppm
inhibiteurs de polymérisation non phénoliques < 10 ppm, en particulier < 3 ppm.

L'installation adaptée pour mettre en oeuvre le procédé de récupération de l'acide acrylique de grade polymère tel que décrit, comprend au moins :
a) une colonne de déshydratation ;
b) une colonne de finition connectée fluidiquement en pied de ladite colonne de déshydratation ;
c) éventuellement au moins une colonne de distillation connectée fluidiquement latéralement à ladite colonne de finition ;
d) un dispositif de mélange assurant l'introduction, le mélange et les conditions optimales de réaction d'un agent de traitement chimique dans le flux de pied de la colonne de déshydratation alimentant la colonne de finition, comprenant éventuellement une capacité intermédiaire ;
e) au moins un système de soutirage latéral pour la colonne de finition.

Comme dispositifs de mélange utilisables, on peut citer un récipient agité ou recirculé, ou un mélangeur en ligne, éventuellement associé à une capacité intermédiaire de stockage. Le dispositif de mélange permet d'assurer un temps de séjour minimum, généralement compris entre 5 min et 120 min, et de fixer une température optimale comprise en général entre 30°C et 80°C, pour que la réaction de l'agent de traitement chimique avec les impuretés notamment de type aldéhydes soit la plus efficace, sans occasionner de dépôt solide.

Un autre objet de l'invention porte sur un procédé de production d'acide acrylique de grade polymère comprenant au moins les étapes suivantes :
A) on soumet à une oxydation en phase gazeuse au moins un précurseur d'acide acrylique pour former un mélange réactionnel gazeux comprenant de l'acide acrylique ;
B) on refroidit le mélange réactionnel gazeux ;
C) on soumet le mélange réactionnel gazeux refroidi au procédé de récupération de l'acide acrylique tel que défini précédemment.

Le précurseur de l'acide acrylique peut être l'acroléine et peut être dérivé de matière première renouvelable produisant ainsi de l'acide acrylique biosourcé.

De préférence, le précurseur de l'acide acrylique est l'acroléine obtenu par oxydation catalytique de propylène.

La réaction d'oxydation de l'étape A), effectuée selon les connaissances de l'art, fournit généralement un mélange réactionnel gazeux, surchauffé à une température supérieure à 280°C.

Ce mélange est avantageusement refroidi selon une étape B), notamment jusqu'à une température inférieure à 250°C, de préférence inférieure à 190°C, pour être soumis selon l'étape C) au procédé de récupération de l'acide acrylique sans utiliser de solvant azéotropique et incluant un traitement chimique d'élimination des aldéhydes. Il peut être refroidi directement dans la colonne de déshydratation, ou peut être refroidi à l'aide d'un échangeur de chaleur situé en amont de la colonne de déshydratation.

Le procédé selon l'invention fournit un acide acrylique de grade polymère (ou AAg) utilisable pour préparer des superabsorbants acryliques destinés aux domaines de l'hygiène ou de la câblerie, ou des esters acryliques de haute pureté, ou encore des dispersants ou floculants acryliques comportant une faible teneur en composés organiques volatils.

L'invention sera maintenant illustrée par les exemples suivants, qui n'ont pas pour but de limiter la portée de l'invention, définie par les revendications annexées.

### PARTIE EXPERIMENTALE

Les pourcentages sont exprimés en pourcentages massiques.

Dans les exemples qui suivent, on a utilisé les méthodes d'analyse suivantes :
- Chromatographie en phase liquide haute performance sur Colonne Atlantis DC18 du fournisseur Waters, avec détection UV et étalonnage externe : furfural, benzaldéhyde, anhydride maléique dosé sous forme d'acide maléique, acide acétique, phénothiazine.
- Spectrométrie UV-visible, après réaction de l'acroléine avec le 4-hexyl resorcinol en milieu éthanol / acide trichloracétique, catalysée par le chlorure mercurique, et développement d'une coloration bleue présentant une absorbance maximale à 603nm : acroléine

### Exemple 1 (comparatif)

Des simulations à l'aide du logiciel ASPEN ont été utilisées pour illustrer un procédé selon l'art antérieur. Il s'agit notamment du procédé décrit dans le brevet EP 2 066 613 B1 qui fournit en soutirage latéral de la colonne 17, un flux 5 d'acide acrylique purifié tel que représenté sur la Figure 3.

Les débits, températures, pressions et compositions en composés principaux des flux sont reportés dans le tableau ci-après :

| N° flux | | 1 | 14/15 | 3 | 11 | 8 | 5 | 6 |
|---|---|---|---|---|---|---|---|---|
| Débit (kg/h) | | 70501 | 59058 | 49800 | 335709 | 37642 | 11000 | 1195 |
| Pression (bar abs) | | 1.36 | 1.12 | 0.12 | 1.40 | 0.12 | 0.21 | 0.24 |
| Température (°C) | | 181 | 59 | 62 | 100 | 73 | 96 | 102 |

| Composition | | % massique | | | | | | |
|---|---|---|---|---|---|---|---|---|
| Composés incondensables | | 73.36% | 87.56% | 0.016% | 0.014% | 0.102% | 0.058% | <0.001% |
| | H2O | 8.51% | 10.83% | 4.40% | 4.56% | 5.86% | 0.00% | 0.00% |
| Acide acrylique | | 16.70% | 0.26% | 85.01% | 84.54% | 80.62% | 99.81% | 84.18% |
| Acide acétique | | 0.56% | 0.65% | 10.05% | 10.41% | 13.29% | 0.05% | <0.001% |
| Furfural | | 0.0051% | <0.001% | 0.0112% | 0.0105% | 0.0052% | 0.0142% | 0.1690% |
| Benzaldéhyde | | 0.0085% | <0.001% | 0.0154% | 0.0142% | 0.0049% | 0.0082% | 0.4141% |
| Acroléine | | 0.1976% | 0.2341% | 0.0280% | 0.0287% | 0.0371% | <0.001% | <0.001% |
| Acide + anhydride maléique | | 0.15% | <0.001% | 0.23% | 0.21% | 0.03% | 0.04% | 7.11% |
| Dimère acide acrylique | | 0.12% | 0.00% | 0.16% | 0.14% | <0.001% | <0.001% | 6.79% |

Dans ce tableau, il apparait que le rapport de concentration massique furfural /acroléine est bien inférieur à 3 dans le flux 3, et la qualité d'acide acrylique purifié obtenue en soutirage latéral de la colonne 17 (flux 5) n'est pas de qualité suffisante pour être utilisée dans une application requérant une qualité d'acide acrylique de grade polymère. En particulier, les concentrations de furfural et benzaldéhyde et acide / anhydride maléique sont trop importantes pour fabriquer des polymères de forte masse moléculaire répondant aux souhaits des applicateurs.

### Exemple 2 (référence)

Un mélange synthétique représentatif du milieu obtenu en pied de colonne de déshydratation d'un procédé de purification d'acide acrylique n'utilisant pas de solvant organique externe est préparé. Ce flux synthétique a la composition suivante : Acide acrylique (85,20%), acide acétique (10%), eau (4,4%), acroléine (0,03%), furfural (0,011%), benzaldéhyde (0,015%), anhydride maléique (0,2%), phénothiazine (0,1%), hydroquinone (0,05%).

250g de ce mélange sont introduits dans un ballon en verre et le mélange est distillé dans un évaporateur rotatif à température de 90°C sous pression de 30 mbar, de façon à recueillir environ 80% de fraction distillée.

Après évaporation, on recueille 201g de distillat incolore et limpide, titrant 0,01% furfural, 0,01% benzaldéhyde, 0,03% acroléine, 0,15% d'acide / anhydride maléique. Les résultats de cet essai reflètent la répartition des impuretés dans les conditions de distillation flash, sans plateau de séparation. Il sert de référence aux essais suivants réalisés en présence d'agents chimiques de traitement des aldéhydes.

### Exemple 3 (selon l'invention)

Le même traitement que l'essai 2 est répété à partir de 200g du même mélange synthétique, mais en ajoutant cette fois 0,35g d'hydrate d'hydrazine. En outre, le mélange est d'abord porté à 40°C pendant 60 minutes sous pression atmosphérique, puis distillé dans l'évaporateur rotatif à température de 90°C sous pression de 30 mbar.

On récupère 154g (77%) de distillat incolore et limpide, titrant < 1ppm furfural, < 1ppm benzaldéhyde, < 1ppm acroléine, < 1ppm d'acide / anhydride maléique.

Le résidu recueilli non distillé recueilli après évaporation est limpide et sans dépôt de solide.

### Exemple 4 (comparatif)

Le même traitement que l'essai 3 est répété, à partir de 200g de mélange synthétique décrit dans l'essai 2, mais en ajoutant cette fois 0,31g de bicarbonate d'aminoguanidine. Après évaporation, on recueille 152g (76%) de distillat incolore et limpide, titrant < 1ppm furfural, < 1ppm benzaldéhyde, < 1ppm acroléine, 48ppm d'acide / anhydride maléique. Le résidu recueilli non distillé recueilli après évaporation est limpide et sans dépôt de solide.

### Exemple 5 (comparatif)

Le même traitement que l'essai 3 est répété, à partir de 200g de mélange synthétique décrit dans l'essai 2, mais en ajoutant cette fois 0,47g de métaphénylènediamine.

Après évaporation, on recueille 157g (78%) de distillat incolore et limpide, titrant < 1ppm furfural, 3ppm benzaldéhyde, < 1ppm acroléine, 7ppm d'acide / anhydride maléique. Le résidu recueilli non distillé recueilli après évaporation est limpide et sans dépôt de solide.

Les exemples 3, 4 et 5 montrent qu'il est possible d'obtenir, à partir d'un flux représentatif de fond de colonne de déshydratation obtenu selon un procédé de récupération / purification sans addition de solvant, une qualité d'acide acrylique de grade polymère, sans étape de distillation supplémentaire, grâce à un traitement par un agent chimique.

### Exemple 6 (référence)

Un mélange synthétique représentatif du milieu obtenu en pied de colonne de déshydratation d'un procédé de purification d'acide acrylique n'utilisant pas de solvant organique est préparé. Ce flux synthétique a la composition suivante : Acide acrylique (84,6%), acide acétique (9,7%), acide formique (0.1%), eau (4,3%), acroléine (0,027%), furfural (0,010%), benzaldéhyde (0,015%), anhydride maléique (0,20%), phénothiazine (0,1%).

Ce mélange synthétique est introduit en mode continu à un débit de 289 g/h dans un réacteur en verre de 500 ml maintenu à niveau constant, chauffé à 50°C, sous agitation constante et vigoureuse. La température régulée dans le réacteur est de 50°C et le temps de séjour du mélange dans cette capacité agitée est de 1 heure.

Le mélange réactionnel sortant du réacteur alimente un rebouilleur à thermosiphon chauffé par résistance électrique.

Dans les conditions de distillation flash opérées dans le rebouilleur, sous une pression de 345 hPa, à température de 101°C, on distille un flux de tête représentant 90% du flux d'alimentation.

L'analyse du flux de tête indique une concentration de 113ppm de furfural, et le flux de pied contient 163ppm de furfural. Aucun encrassement notoire n'est observé.

Cet exemple montre que, en l'absence d'additif chimique réagissant avec les aldéhydes, le furfural se partage entre le flux de tête et le flux de pied, et la qualité du flux de tête n'est pas satisfaisante.

### Exemple 7 (selon l'invention)

L'expérience décrite dans l'exemple 6 est refaite dans les mêmes conditions, mais en présence d'un agent de traitement d'élimination des aldéhydes.

Le mélange synthétique de composition identique à celle de l'exemple 6 est introduit en mode continu à un débit de 235g/h dans le réacteur chauffé à 50°C. On introduit également dans ce réacteur 0,78g/h d'hydrate d'hydrazine. Le temps de séjour du mélange dans le réacteur est égal à une heure. La quantité d'agent chimique ajouté correspond à un rapport molaire de 2 par rapport à la somme des aldéhydes présents dans le milieu à traiter Le mélange réactionnel sortant du réacteur alimente le rebouilleur à thermosiphon sous une pression de 276 hPa. Suite à la distillation flash, on obtient après condensation 219g/h de distillat liquide (93% du flux alimentant le bouilleur) et 16 g/h de flux de pied. Après analyse du flux de tête, la concentration de furfural (impureté la plus difficile à séparer parce que la plus proche de l'acide acrylique en termes de température d'ébullition), est de 1ppm.

Dans ce mélange, le ratio de la concentration de furfural / acroléine dans le flux d'alimentation est de 0,4. Bien que ce ratio soit situé nettement en dehors de l'intervalle de 3 à 100 spécifié dans le brevet EP1110940 comme étant optimal, cet exemple montre que l'élimination du furfural est efficace. Par ailleurs, aucun encrassement notoire n'est observé dans le montage.

### Exemple 8 (comparatif)

L'expérience décrite dans l'exemple 7 est refaite dans les mêmes conditions, mais l'agent de traitement chimique introduit dans le réacteur est une solution à 20% de bicarbonate d'aminoguanidine préparée dans l'acide acrylique glacial (exempt d'impuretés aldéhydes). Le mélange synthétique de composition identique à celle de l'exemple 7 (235 g/h) et la solution d'agent chimique de traitement des aldéhydes (11,8 g/h) sont introduits sous agitation vigoureuse dans le réacteur maintenu à 50°C, avec un temps de séjour d'une heure. La quantité d'agent chimique ajouté correspond à un rapport molaire de 2,2 par rapport à la somme des aldéhydes présents dans le milieu à traiter. Le mélange réactionnel sortant du réacteur est envoyé en alimentation du rebouilleur, opéré à température de 101°C sous 315 hPa. On distille un flux de tête correspondant à 84% du flux d'alimentation.

Après analyse, le flux de tête contient 1 ppm de furfural. Aucun encrassement significatif de l'équipement n'est observé.

## Revendications

1. Procédé de récupération d'acide acrylique de grade polymère ayant une teneur en poids en acide acrylique supérieure à 99,5 %, une teneur en aldéhydes totaux inférieure à 3 ppm et les teneurs en impuretés suivantes : protoanémonine < 5 ppm, anhydride maléique < 100 ppm et inhibiteurs de polymérisation non phénoliques < 10 ppm, en l'absence de solvant organique, à partir d'un mélange réactionnel gazeux comprenant de l'acide acrylique obtenu par oxydation en phase gazeuse d'un précurseur de l'acide acrylique, comprenant au moins les étapes suivantes :
i) on soumet le mélange réactionnel gazeux à une déshydratation sans utiliser de solvant azéotropique dans une première colonne de distillation dite colonne de déshydratation, conduisant à un flux de tête dont une partie au moins est condensée et renvoyée à la colonne de déshydratation sous forme de reflux, et à un flux de pied ;
ii) on soumet au moins en partie le flux de pied de colonne de déshydratation à une distillation dans une seconde colonne dite colonne de finition, conduisant à un flux de tête, et à un flux de pied contenant des composés lourds ;
iii) on récupère un flux d'acide acrylique purifié de grade polymère par soutirage latéral de la colonne de finition ;
**caractérisé en ce qu'**il est mis en oeuvre en présence d'un agent de traitement chimique des aldéhydes visant à réduire, jusqu'à un niveau très faible le taux d'aldéhydes, ledit agent chimique réagissant avec les aldéhydes présents pour former des produits lourds séparables de l'acide acrylique, ledit agent de traitement chimique des aldéhydes étant choisi parmi l'hydrazine et ses sels, ledit agent de traitement chimique des aldéhydes étant introduit à l'étape ii).

2. Procédé selon la revendication 1 **caractérisé en ce que** la colonne de finition est une colonne de distillation classique opérant à une pression inférieure à la pression atmosphérique.

3. Procédé selon la revendication 1 **caractérisé en ce que** la colonne de finition est une colonne à paroi séparatrice alimentée d'un côté de la paroi par le flux de pied de colonne de déshydratation et le soutirage latéral du flux d'acide acrylique purifié est réalisé dans la section située de l'autre côté de la paroi séparatrice.

4. Procédé selon l'une quelconque des revendications 1 à 3 **caractérisé en ce que** l'agent de traitement chimique des aldéhydes est introduit dans la colonne de finition au niveau d'une section située entre le niveau d'alimentation de ladite colonne et le niveau du plateau de soutirage latéral de l'acide acrylique purifié.

5. Procédé selon l'une quelconque des revendications 1 à 3 **caractérisé en ce que** l'agent de traitement chimique des aldéhydes est introduit dans la colonne de finition par l'intermédiaire du flux d'alimentation de ladite colonne.

6. Procédé selon l'une quelconque des revendications 1 à 3 **caractérisé en ce que** l'agent de traitement chimique des aldéhydes est introduit en pied de la colonne de déshydratation, de préférence dans le flux liquide amené en contact avec le flux réactionnel en vue de son refroidissement.

7. Procédé selon l'une quelconque des revendications 1 à 6 **caractérisé en ce que** le flux d'acide acrylique purifié obtenu à l'étape iii) est soutiré sous forme d'un flux gazeux d'acide acrylique de grade polymère.

8. Procédé selon l'une quelconque des revendications précédentes **caractérisé en ce que** l'agent de traitement chimique des aldéhydes est introduit par l'intermédiaire d'un dispositif de mélange comprenant au moins une capacité assurant le mélange intime de l'agent chimique avec le flux à traiter à une température comprise entre 30°C et 80°C, durant un temps de séjour allant de 5 min à 120 min.

9. Procédé selon l'une quelconque des revendications précédentes **caractérisé en ce que** l'agent de traitement chimique des aldéhydes est choisi parmi : l'hydrate d'hydrazine, le sulfate d'hydrazine, les carboxylates d'hydrazine, le chlorhydrate d'hydrazine, la phénylhydrazine, la 4-nitrophénylhydrazine, et la 2,4-dinitrophénylhydrazine, seuls ou leurs mélanges en toutes proportions.

10. Procédé selon l'une quelconque des revendications précédentes caractérisé en ce l'on opère un second soutirage en phase gazeuse sur la colonne de finition, à une position située en dessous du soutirage latéral de l'acide acrylique purifié.

11. Procédé selon l'une quelconque des revendications précédentes **caractérisé en ce que** le précurseur de l'acide acrylique est l'acroléine, obtenue par oxydation de propylène ou par oxydéshydrogénation de propane.

12. Procédé selon l'une quelconque des revendications 1 à 10 **caractérisé en ce que** le précurseur de l'acide acrylique comprend du carbone d'origine renouvelable, et est dérivé du glycérol, de l'acide 3-hydroxypropionique ou de l'acide 2-hydroxypropionique.

13. Procédé de production d'acide acrylique de grade polymère ayant une teneur en poids en acide acrylique supérieure à 99,5 %, une teneur en aldéhydes totaux inférieure à 3 ppm et les teneurs en impuretés suivantes : protoanémonine < 5 ppm,
anhydride maléique < 100 ppm et inhibiteurs de polymérisation non phénoliques < 10 ppm, comprenant au moins les étapes suivantes :
A) on soumet à une oxydation en phase gazeuse au moins un précurseur d'acide acrylique pour former un mélange réactionnel gazeux comprenant de l'acide acrylique ;
B) on refroidit le mélange réactionnel gazeux ;
C) on soumet le mélange réactionnel gazeux refroidi au procédé de récupération de l'acide acrylique tel que défini à l'une quelconque des revendications 1 à 12.

## Patentansprüche

1. Verfahren zur Gewinnung von Acrylsäure der Polymerklasse mit einem gewichtsbezogenen Gehalt an Acrylsäure von mehr als 99,5 %, einem Gehalt an Gesamtaldehyden von weniger als 3 ppm und den folgenden Gehalten an Verunreinigungen: Protoanemonin < 5 ppm, Maleinsäureanhydrid < 100 ppm und nicht-phenolische Polymerisationsinhibitoren < 10 ppm, ohne organische Lösemittel, ausgehend von einem gasförmigen Reaktionsgemisch, das Acrylsäure umfasst, die durch Gasphasenoxidation eines Vorläufers der Acrylsäure erhalten wird, umfassend mindestens die folgenden Schritte:
i) das gasförmige Reaktionsgemisch wird ohne Einsatz eines azeotropen Lösemittels einer Dehydrierung in einer ersten Destillationskolonne, Dehydrierungskolonne genannt, unterzogen, was zu einem Kopfstrom, von dem mindestens ein Teil kondensiert wird und zu der Dehydrierungskolonne in Form eines Rückflusses zurückgeleitet wird, und zu einem Sumpfstrom führt;
ii) der Sumpfstrom der Dehydrierungskolonne wird mindestens zum Teil einer Destillation in einer zweiten Kolonne, Abschlusskolonne genannt, unterzogen, was zu einem Kopfstrom und zu einem schwerflüchtige Verbindungen enthaltenden Sumpfstrom führt;
iii) ein gereinigter Acrylsäurestrom der Polymerklasse wird durch seitliches Abziehen aus der Abschlusskolonne gewonnen;
**dadurch gekennzeichnet, dass** es in Gegenwart eines Mittels zur chemischen Behandlung der Aldehyde durchgeführt wird, was darauf abzielt, den Aldehydanteil bis auf ein sehr geringes Niveau zu reduzieren, wobei das chemische Mittel mit den vorhandenen Aldehyden reagiert, um schwerflüchtige Stoffe zu bilden, die von der Acrylsäure abtrennbar sind, wobei das Mittel zur chemischen Behandlung der Aldehyde aus Hydrazin und seinen Salzen ausgewählt ist, wobei das Mittel zur chemischen Behandlung im Schritt ii) eingeleitet wird.

2. Verfahren nach Anspruch 1, **dadurch gekennzeichnet, dass** die Abschlusskolonne eine klassische Destillationskolonne ist, die bei einem geringeren Druck als dem atmosphärischen Druck arbeitet.

3. Verfahren nach Anspruch 1, **dadurch gekennzeichnet, dass** die Abschlusskolonne eine Trennwandkolonne ist, der auf einer Seite der Wand der Sumpfstrom der Dehydrierungskolonne zugeführt wird, und das seitliche Abziehen des gereinigten Acrylsäurestroms in dem auf der anderen Seite der Trennwand gelegenen Abschnitt ausgeführt wird.

4. Verfahren nach einem der Ansprüche 1 bis 3, **dadurch gekennzeichnet, dass** das Mittel zur chemischen Behandlung der Aldehyde in die Abschlusskolonne auf Höhe eines Abschnitts eingeleitet wird, der zwischen der Zuführungshöhe der Kolonne und der Höhe des Bodens zum seitlichen Abziehen der gereinigten Acrylsäure gelegen ist.

5. Verfahren nach einem der Ansprüche 1 bis 3, **dadurch gekennzeichnet, dass** das Mittel zur chemischen Behandlung der Aldehyde in die Abschlusskolonne über den Feedstrom der Kolonne eingeleitet wird.

6. Verfahren nach einem der Ansprüche 1 bis 3, **dadurch gekennzeichnet, dass** das Mittel zur chemischen Behandlung der Aldehyde in den Sumpf der Dehydrierungskolonne eingeleitet wird, bevorzugt in den flüssigen Strom, der zu dessen Kühlung in Kontakt mit dem Reaktionsstrom gebracht wird.

7. Verfahren nach einem der Ansprüche 1 bis 6, **dadurch gekennzeichnet, dass** der im Schritt iii) erhaltene gereinigte Acrylsäurestrom in Form eines gasförmigen Acrylsäurestroms der Polymerklasse abgezogen wird.

8. Verfahren nach einem der vorhergehenden Ansprüche, **dadurch gekennzeichnet, dass** das Mittel zur chemischen Behandlung der Aldehyde über eine Mischvorrichtung eingeleitet wird, die mindestens eine Kapazität umfasst, die das innige Mischen des chemischen Mittels mit dem zu behandelnden Strom bei einer Temperatur zwischen 30 °C und 80 °C während einer Verweilzeit von 5 min bis 120 min gewährleistet.

9. Verfahren nach einem der vorhergehenden Ansprüche, **dadurch gekennzeichnet, dass** das Mittel zur chemischen Behandlung der Aldehyde ausgewählt ist aus: Hydrazinhydrat, Hydrazinsulfat, den Hydraxincarboxylaten, Hydrazinchlorhydrat, Phenylhydrazin, 4-Nitrophenylhydrazin und 2,4-Dinitrophenylhydrazin, allein oder als Gemisch in beliebigen Verhältnissen.

10. Verfahren nach einem der vorhergehenden Ansprüche, **dadurch gekennzeichnet, dass** ein zweites Abziehen in gasförmiger Phase an der Abschlusskolonne an einer Stelle vorgenommen wird, die unter dem Seitenabzug für die gereinigte Acrylsäure gelegen ist.

11. Verfahren nach einem der vorhergehenden Ansprüche, **dadurch gekennzeichnet, dass** der Vorläufer der Acrylsäure das Acrolein ist, das durch Oxidation von Propylen oder durch Oxydehydrierung von Propan erhalten wird.

12. Verfahren nach einem der Ansprüche 1 bis 10, **dadurch gekennzeichnet, dass** der Vorläufer der Acrylsäure Kohlenstoff erneuerbaren Ursprungs umfasst und aus Glycerol, 3-Hydroxypropionsäure oder 2-Hydroxypropionsäure abgeleitet ist.

13. Verfahren zur Herstellung von Acrylsäure der Polymerklasse mit einem gewichtsbezogenen Gehalt an Acrylsäure von mehr als 99,5 %, einem Gehalt an Gesamtaldehyden von weniger als 3 ppm und den folgenden Gehalten an Verunreinigungen: Protoanemonin < 5 ppm, Maleinsäureanhydrid < 100 ppm und nicht-phenolische Polymerisationsinhibitoren < 10 ppm, umfassend mindestens die folgenden Schritte:
A) mindestens ein Vorläufer von Acrylsäure wird einer Gasphasenoxidation unterzogen, um ein gasförmiges Reaktionsgemisch zu bilden, das Acrylsäure umfasst;
B) das gasförmige Reaktionsgemisch wird gekühlt;
C) das gekühlte gasförmige Reaktionsgemisch wird dem Verfahren zur Gewinnung von Acrylsäure gemäß der Definition in einem Ansprüche 1 bis 12 unterzogen.

## Claims

1. Process for the recovery of polymer-grade acrylic acid having a content by weight of acrylic acid higher than 99.5%, comprising a content of total aldehydes < 3 ppm, and the contents of following impurities: protoanemonin < 5 ppm, maleic anhydride < 100 ppm, non-phenolic polymerization inhibitors < 10 ppm, in the absence of organic solvent, from a gaseous reaction mixture comprising acrylic acid obtained by gas-phase oxidation of a precursor of the acrylic acid, comprising at least the following stages:
i) the gaseous reaction mixture is subjected to dehydration without using azeotropic solvent in a first distillation column, referred to as dehydration column, resulting in a top stream, at least a portion of which is condensed and sent back to the dehydration column in the form of reflux, and in a bottom stream;
ii) the dehydration column bottom stream is subjected, at least in part, to distillation in a second column, referred to as finishing column, resulting in a top stream and in a bottom stream comprising heavy compounds;
iii) a purified (meth)acrylic acid stream of polymer grade is recovered by drawing off a sidestream from the finishing column;
**characterized in that** it is carried out in the presence of at least one agent for the chemical treatment of the aldehydes which makes it possible to reduce, down to a very low level, the content of the aldehydes, said chemical treatment agent reacting with the aldehydes present to form heavy reaction products which can be more easily separated from the acrylic acid, said chemical treatment agent reacting with the aldehydes being selected from hydrazine and its salts, said chemical treatment agent reacting with the aldehydes being introduced at step ii).

2. Process according to Claim 1, **characterized in that** the finishing column is a conventional distillation column operating at a pressure below atmospheric pressure.

3. Process according to Claim 1, **characterized in that** the finishing column is a column having a separating wall fed, on one side of the wall, with the dehydration column bottom stream and the drawing off of a sidestream of the purified acrylic acid stream is carried out in the section located on the other side of the separating wall.

4. Process according to any one of Claims 1 to 3, **characterized in that** the agent for the chemical treatment of the aldehydes is introduced into the finishing column at the level of a section located between the feed level of the said column and the level of the plate for drawing off a sidestream of purified acrylic acid.

5. Process according to any one of Claims 1 to 3, **characterized in that** the agent for the chemical treatment of the aldehydes is introduced into the finishing column via the feed stream of the said column.

6. Process according to any one of Claims 1 to 3, **characterized in that** the agent for the chemical treatment of the aldehydes is introduced at the bottom of the dehydration column, preferably in the liquid stream brought into contact with the reaction stream for the purpose of the cooling thereof.

7. Process according to any one of Claims 1 to 6, **characterized in that** the purified acrylic acid stream obtained in stage iii) is drawn off in the form of a gaseous stream of polymer-grade acrylic acid.

8. Process according to any one of the preceding claims, **characterized in that** the agent for the chemical treatment of the aldehydes is introduced via a mixing device comprising at least one tank ensuring the intimate mixing of the chemical agent with the stream to be treated at a temperature of between 30°C and 80°C for a residence time ranging from 5 min to 120 min.

9. Process according to any one of the preceding claims, **characterized in that** the agent for the chemical treatment of the aldehydes is chosen from: hydrazine hydrate, hydrazine sulphate, hydrazine carboxylates, hydrazine hydrochloride, phenylhydrazine, 4-nitrophenylhydrazine, and 2,4-dinitrophenylhydrazine, alone or their mixtures in all proportions.

10. Process according to any one of the preceding claims, **characterized in that** a second drawing off is carried out in the gas phase on the finishing column, at a position located below the drawing off of a sidestream of the purified acrylic acid.

11. Process according to any one of the preceding claims, **characterized in that** the precursor of the acrylic acid is acrolein, obtained by oxidation of propylene or by oxydehydrogenation of propane.

12. Process according to any one of Claims 1 to 10, **characterized in that** the precursor of the acrylic acid comprises carbon of renewable origin and is derived from glycerol, 3-hydroxypropionic acid or 2-hydroxypropionic acid.

13. Process for the production of polymer-grade acrylic acid having a content by weight of acrylic acid higher than 99.5%, comprising a content of total aldehydes < 3 ppm, and the contents of following impurities: protoanemonin < 5 ppm, maleic anhydride < 100 ppm, non-phenolic polymerization inhibitors < 10 ppm, comprising at least the following stages:
A) at least one acrylic acid precursor is subjected to gas-phase oxidation in order to form a gaseous reaction mixture comprising acrylic acid;
B) the gaseous reaction mixture is cooled;
C) the cooled gaseous reaction mixture is subjected to the process for the recovery of acrylic acid as defined in any one of Claims 1 to 12.
